# EUROPÄISCHE PATENTSCHRIFT

(11) **EP 0 581 836 B1**
(45) Veröffentlichungstag und Bekanntmachung des Hinweises auf die Patenterteilung: **18.11.1998**
(21) Anmeldenummer: 92909457.1
(22) Anmeldetag: 24.04.1992
(51) Int. Cl.: C12N 15/90, C12N 15/74, C12N 15/63, A61K 39/10, C12N 15/31, C12N 1/21

(54) **HYBRIDTRANSPOSON MIT PT-OPERON, PLASMIDE UND BAKTERIENSTÄMME DAFÜR**
HYBRID TRANSPOSON WITH A PT-OPERON, PLASMIDS AND BACTERIAL STRAINS THEREFOR
TRANSPOSON HYBRIDE CONTENANT UN OPERON DE LA TOXINE DE PERTUSSIS, PLASMIDES ET SOUCHES BACTERIENNES POUR SON OBTENTION

(30) Priorität: 24.04.1991 DE 4113385
(43) Veröffentlichungstag der Anmeldung: 09.02.1994
(73) Patentinhaber: Gesellschaft für biotechnologische Forschung mbH (GBF), 38124 Braunschweig (DE)
(72) Erfinder: WALKER, Mark, D-3300 Braunschweig (DE); TIMMIS, Kenneth, D-3300 Braunschweig (DE)
(74) Vertreter: Boeters, Hans Dietrich, Dr.
(86) Internationale Anmeldenummer: EP9200910
(87) Internationale Veröffentlichungsnummer: WO9219750

(56) Entgegenhaltungen:
- EP-A- 0 396 964
- US-A- 4 883 761
- INFECTION & IMMUNITY, vol. 59, no. 11, November 1991, Washington, DC (US); M.J. WALKER et al., pp. 4238-4248
- JOURNAL OF BACTERIOLOGY, vol. 172, no. 11, November 1990, Baltimore, MD (US); M. HERRERO et al., pp. 6557-6567
- INFECTION & IMMUNITY, vol. 57, no. 5, May 1989, Washington, DC (US); C.K. LEE et al., pp. 1413-1418
- GENE, vol. 51, 1987, Amsterdam (NL); M.G. OBUKOWICZ et al., pp. 91-96

## Beschreibung

Wachsende Bedenken gegen Nebenwirkungen, die mit einer Immunisierung bei der Verwendung von Gesamtzellzubereitungen von *B. pertussis* verbunden sind, haben zu einer abnehmenden Akzeptanz von Impfstoffen gegen Keuchhusten geführt. Das hat wiederum erhöhtes Auftreten dieser Krankheit mit sich gebracht. Es ist unumgänglich, daß ein wirksamer, nicht-reaktogener Impfstoff hoher Akzeptanz entwickelt wird. Verschiedene möglicherweise schützende Antigene sind Kandidaten für eine Aufnahme in einen gereinigten Mehrkomponenten-Impfstoff unter Einschluß von Toxinen, wie Pertussis-Toxin (PT) und Adenylat-Cyclase, ferner auch verschiedene Antigene der Zelloberfläche oder sekretierte Antigene, wie filamentöses Hämagglutinin und serotypspezifische Fimbriae (15, 16). *Bordetella pertussis* ist jedoch hinsichtlich seiner Ernährung ein heikler Mikroorganismus. Die Reinigung von direkt aus *B. pertussis* gewonnenen Antigenen wird durch antigene Variation, die von dem Bvg-positiven Regulationslocus (5, 9, 18) kontrolliert wird, geringen bakteriellen Wachstumsraten, niedrigen Ausbeuten und die Anwesenheit von reaktogenen Verunreinigungen behindert. Um diese Nachteile zu überwinden, sind Anstrengungen unternommen worden, eine Expression von rekombinanten Antigenen in E. coli mit starken Transkriptions- und Translationssignalen zu erreichen. Die fünf Gene, die PT kodieren, sind unäbhängig voneinander in E. coli unter der Kontrolle des Lambda-Promotors P_{L} exprimiert worden (2). Die fimbriale Untereinheit vom Serotyp 2 ist gleichfalls mit Hilfe der lambda-Promotoren P_{L} und P_{R} exprimiert worden (17). In beiden Fällen ließen sich die rekombinanten Proteine nicht zu Produkten zusammenführen, die immunologisch mit den nativen Antigenen identisch waren. Pertussis-Holotoxin kann in rekombinanten Stämmen von *B. parapertussis* und *B. bronchiseptica* unter der Kontrolle des ursprünglichen *B.-pertussis-Promotors* gebildet werden. Jedoch fand man einen Expressionsgrad, der geringer als der bei *B. pertussis* vom Wildtyp war. Auch unterlag die Expression einer Modulation und einer Bvg-kontrollierten Phasenvariation. Rekombinante und für einen breiten Bereich von Wirten einsetzbare Plasmide, die das PT-Operon trugen, waren gleichfalls außerordentlich instabil und unterlagen sowohl einem Plasmidverlust als auch Gendeletionen (11).

Aus J. Bacterioloy, 172 (1990) 6557-6567 ist ferner ein Verfahren für die Klonierung und die stabile Insertion von Genen in Gram-negativen Bakterien bekannt, bei dem Hybridtransposons verwendet werden. Als Beispiel wird die Insertion des Kanamycinresistenzgens in *B. putida* und ferner die Insertion des Melaningens in *K. pneumoniae* angegeben. Hybridtransposons mit einem PT-Operon sind diesem Stand der Technik nicht zu entnehmen.

Die im folgenden beschriebene Erfindung geht von dem vorstehend genannten Stand der Technik sowie von der Aufgabe aus, Pertussis-Toxin zu einer zentralen Komponente einer nenen Generation von Impfstoffen zu machen.

So betrifft eine Ausführungsform der Erfindung ein Hybridtransposon, das dadurch herstellbar ist, daß man zwischen die Umkehrwiederholungen (inverted repeats) eines Transposons ein Pertussis-Toxin-Operon (PT-Operon) einsetzt.

Dieses Hybridtransposon ist also promotorlos. Zur Expression soll es nach chromosomalem Einbau unter der Kontrolle eines nativen Promotors stehen.

Das erfindungsgemäße Hybridtransposon kann dadurch herstellbar sein, daß man die Umkehrwiederholungen ungekürzt oder gekürzt verwendet, wobei die gekürzten Umkehrwiederholungen noch eine Transponierung gestatten.

Das erfindungsgemäße Hybridtransposon kann dadurch gekennzeichnet sein, daß es sich um ein Mini-Transposon handelt.

Das erfindungsgemäße Hybridtransposon kann ferner dadurch herstellbar sein, daß man von Tn5 ausgeht und beispielsweise Umkehrwiederholungen einer Länge von jeweils etwa 19 Basenpaaren übernimmt, wie beispielsweise für TnPtacPT oder TnfusPT.

Das erfindungsgemäße Hybridtransposon kann ferner dadurch herstellbar sein, daß man ein PT-Operon einbaut, bei dem es sich um ein PT-Operon vom Wildtyp handelt oder um ein PT-Operon, das eine Pertussis-Toxin-Modifikation kodiert, die zur Keuchhustenbekämpfung einsetzbar ist.

Das erfindungsgemäße Hybridtransposon kann ferner dadurch gekennzeichnet sein, daß das Hybridtransposon ein Resistenzgen umfaßt, beispielsweise das Kanamycinresistenzgen (Km^{r} = Kan).

Dabei kann das Resistenzgen in derselben oder entgegengesetzter Leserichtung wie das PT-Operon angeordnet sein.

Eine weitere Ausführungsform der Erfindung betrifft ein Plasmid, gekennzeichnet durch ein erfindungsgemäßes Hybridtransposon.

Schließlich betrifft eine Ausführungsform der Erfindung einen Bakterienstamm (a), wie Bordetella pertussis, Bordetella parapertussis oder Bordetella bronchiseptica, beispielsweise ATCC10580, mit einem erfindungsgemäßen Vektor (Plasmid) oder (b) gewinnbar durch Einbau eines PT-Operons mit Hilfe eines erfindungsgemäßen Hybridtransposons oder erfindungsgemäßen Vektors (Plasmids) in Bordetella pertussis, Bordetella parapertussis oder Bordetella bronchiseptica, beispielsweise ATCC10580.

Dabei kann es sich um einen Bvg-negativen Stamm handeln.

Gemäß einer speziellen Ausführungsform der Erfindung werden Mini-Transposons für die konstitutive und induzierbare Expression von PT in Bvg-negativen Stämmen von *B. bronchiseptica* vorgesehen. Dadurch wird eine stabile Hochleistungsexpression von PT in Bvg-negativen *B.-bronchiseptica-*Stämmen möglich gemacht. Die Verwendung derartiger Stämme bietet für die Gewinnung von PT für Impfzwecke eine Reihe von Vorteilen, wie rasche bakterielle Wachstumsraten, hohe Ausbeuten und die Abwesenheit von anderen Bvg-kontrollierten virulenten Determinanten, die bei den angesprochenen Nebenwirkungen von PT-Präparaten bzw. Pertussis-Impfstoffen eine Rolle spielen.

In diesem Zusammenhang ist darauf hinzuweisen, daß unlängst verschiedentlich Methoden für eine genetische Entgiftung von PT entwickelt worden sind (8, 14). Diese Methoden beruhen jedoch auf der Reinigung von entgiftetem PT aus B. pertussis. Die Gewinnung von Pertussis-Holotoxin mit Hilfe von Bvg-negativen Stämmen von *B. bronchiseptica* in Kombination mit Methoden für die genetische Entgiftung von PT wird nun zu einer hochreinen und wenig reaktogenen Impfstoffkomponente führen.

Nachstehend wird die Erfindung mit Figuren und einer Tabelle näher erläutert. Es zeigen:
Figur 1 Konstruktion eines Suicidvektors mit Mini-Transposons für eine PT-Expression unter der Kontrolle des tac-Promotors (pMW126) und als Transkriptonsfusionen mit nativen Promotoren (pMW127).
Figur 2: Southern-Hybridisieranalyse von *B. bronchiseptica* ATCC10580 mit einem Gehalt an PT-exprimierenden Transposons.
Figur 3: Analyse einer PT-Expression von *B. bronchiseptica* ATCC10580 (Streifen 1) mit einem Gehalt an TnPtacPT (Streifen 2) und TnfusPT (Streifen 3), wobei auch *B. pertussis* Tohama (Streifen 4) und gereinigtes PT (Streifen 5) gezeigt sind.

Es wurde vom Plasmid pTX42 (11; vgl auch US-A-4 883 761; ATCC 67046)ausgegangen, das die genetische Information zum Kodieren für die fünf Pertussis-Toxin-Untereinheiten enthält. Ferner ließ man sich von der bekannten Konstruktion von Mini-Transposons (4, 6) für die Expression in Bvg-negativen Stämmen von *B. bronchiseptica* ATCC10580 leiten. Da jedoch die Transposase mit dem Suicidvehikel (suicide delivery vehicle) verloren geht, kann das Mini-Transposon-System nur eine einzige Transpositionsstufe liefern, so daß weitere transpositions- und transposon-bedingte Deletionen und Umlagerungen nicht eintreten.

### Beispiel 1:

### Konstruktion der Mini-Hybrid-Transposons TnPtacPT und TnfusPT und ihr Einbau in das Chromosom von B. bronchiseptica ATCC10580

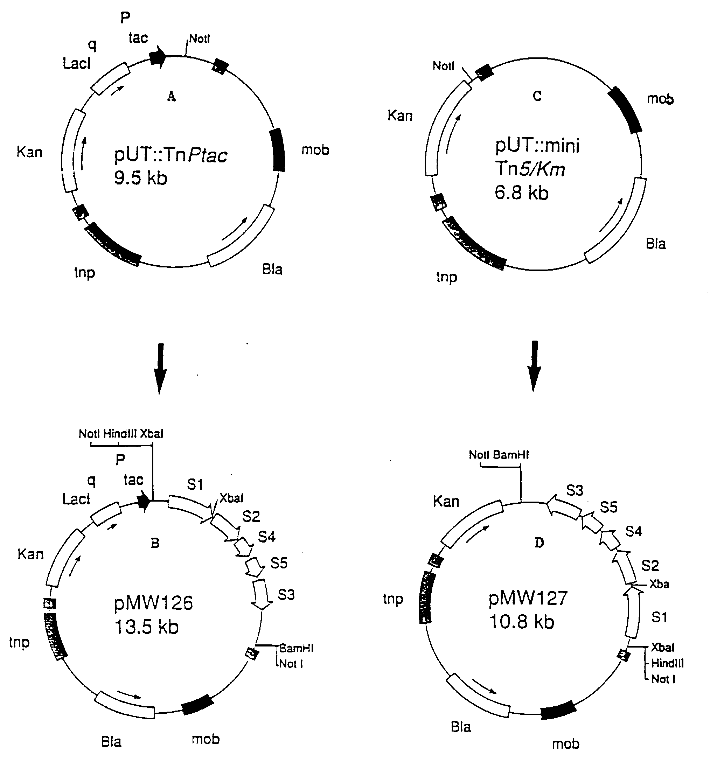

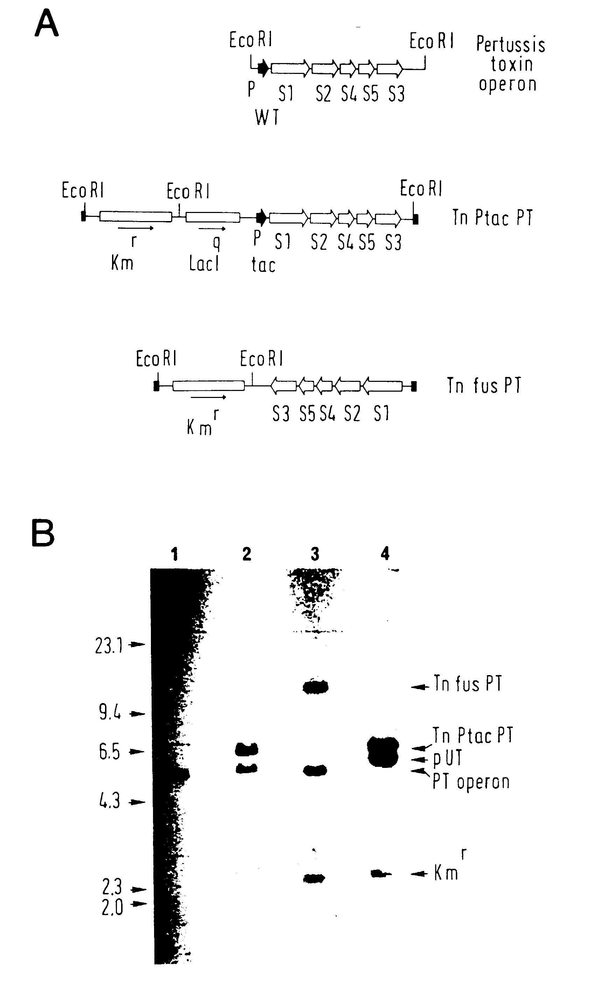

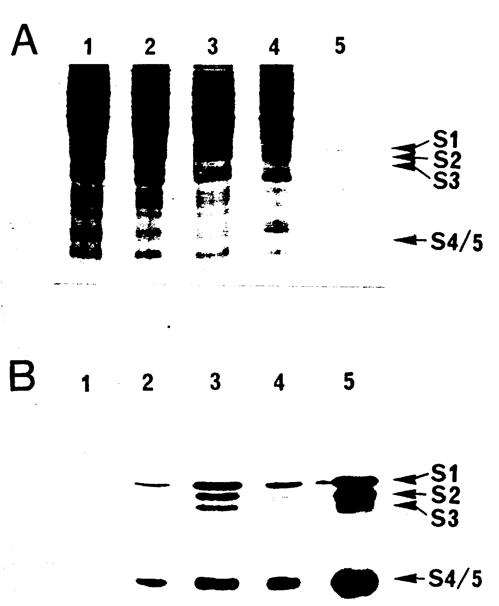

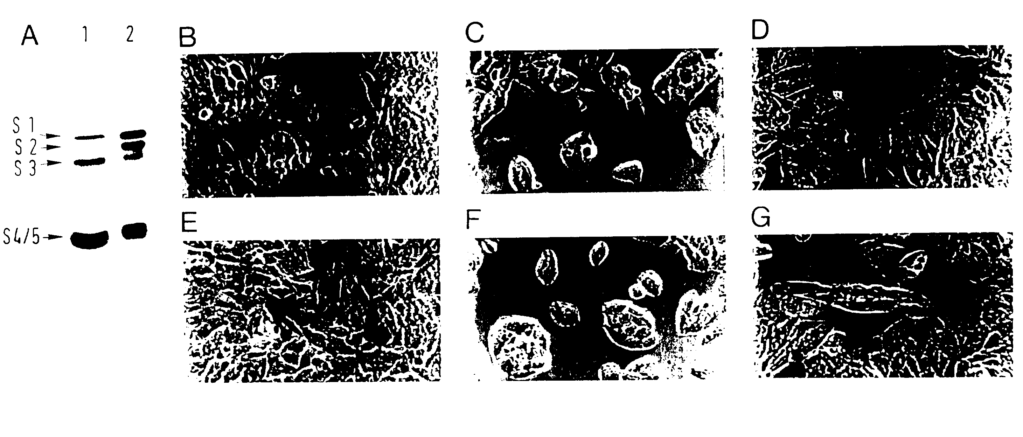

Im Hinblick auf eine Überexpression von PT in dem Bvg-negativen B.-bronchiseptica-Stamm ATCC10580 wurden Mini-Transposons konstruiert, die das PT-Operon enthielten. Das PT-Operon wurde unter Verwendung von Polymerase-Kettenreaktionstechniken in das chromosomale Integrationssystem pUT::miniTn5 kloniert. Das promotorlose PT-Operon wurde (ohne P_{WT})
- einerseits in derselben Orientierung wie der tac-Promotor (Ptac) von pUT::TnPtac unter Bildung von pMW126 (TnPtacPT) und
- andererseits in entgegengesetzter Orientierung in pUT::mini-Tn5/Km unter Bildung von pMW127 (TnfusPT) kloniert (**Figur 1**). Nach der Überführung (mobilization) von pMW126 und pMW127 in ATCC10580 wurden die Transkonjugate im Hinblick auf eine PT-Bildung (Kolonie-Immunoblots; Western-Blots) und auf einen Verlust des Suicidvektors durch Southern Hybridizierung gescreent (**Figur 2**). Selektierte Transkonjuganden erhielten die Bezeichnungen ATCC10580::TNPtacPT bzw. ATCC10580::TnfusPT.

Im Vergleich mit einer PT-Expression von *B. pertussis* lieferte TnPtac eine IPTG-induzierbare PT-Expression auf einem niedrigeren Niveau. Andererseits lieferte TnfusPT eine PT-Expression auf beträchtlich höherem Niveau als *B. pertussis*, und zwar nach transkriptionaler Fusion zwischen einem nativen Promotor (von *B. bronchiseptica*) und dem promotorlosen PT-Operon (**Figur 3**). Im Vergleich zu einem Plasmid (pMW124), das als zusätzliches Merkmal zu denen des für einen breiten Wirtsbereich geeigneten Plasmids pDSK519 (7) das PT-Operon unter der Kontrolle des lac-Promotors enthielt, war das Mini-Transposon-System sehr stabil, ohne daß ein Verlust nach 60 Generationen ohne antibiotische Selektion ermittelt werden konnte. Dazu sei auf die folgende Tabelle 1 verwiesen.

**Tabelle 1:**

| Stabilität der PT-Expression von *B. bronchiseptica* ATCC10580 pMW124 und ATCC10580::TnfusPT nach 60 Generationen ohne antibiotische Selektion. | | | | |
|---|---|---|---|---|
| Bakterienstamm | CFU/ml^{a} | | PT(%) ohne Selektion^{b} | Verknüpfung von PT mit Km^{r} (%)^{c} |
| | Cp^{r} | Cp^{r} Km^{r} | | |
| ATCC10580 pMW124 | 2.1 X 10⁹ | 3.0 x 10⁵ | <1% | 100% |
| ATCC10580::TnfusPT | 3.0 x10⁹ | 2.9 x 10⁹ | 100% | 100% |

| | | | | |
|---|---|---|---|---|
| a: Nach 60 Generationen bestimmte man ohne kanamyzinselektion koloniebildende Einheiten (CFU) pro ml Kultur, indem man entweder mit Cephalexin (Cp) allein oder in Kombination mit Kanamyzin (Km) selektionierte. | | | | |
| b: 100 Cp^{r}-Kolonien wurden hinsichtlich einer PT-Bildung durch Kolonie-Immunoblots gescreent. | | | | |
| c: 100 Cp^{r}-Km^{r}-Kolonien wurden hichsichtlich einer PT-Produktion durch Kolonie-Immunoblots gescreent. | | | | |

Man unterwarf periplasmatische Extrakte dieses Stammes einer Chromatographie an Heparin Sepharose CL6B gemaß FEMS Microbiol. Lett, 51 (1988) 159-162. Im Vergleich zu Pertussis-Holotoxin enthielten diese Präparate erhöhte Mengen der PT-Untereinheiten S3 und S4 (**Figur 4 A.**).

Infect Immun., 57 (1989) 3324-3330 kann man entnehmen, daß das S3-S4-Dimere einen Zuckerbindungsrezeptor enthält. Die Gewichtigkeit von S3 und S4 könnte eine Verknüpfung des S3-S4-Dimeren an Heparin, ein lineares Glucosaminoglykan, widerspiegeIn (12). Auch S1, S2 und S5 konnten von der Heparin-Säule eluiert werden. Danach wurden rekombinante PT-Präparate auf ihre biologische Aktivität mit Hilfe eines CHO-Cell-Cluster-Assay untersucht. Der monoklonale Antikörper E19, der spezifisch an die PT-Untereinheit S1 bindet und ein Klustern von CHO-Zellen durch PT inhibiert, wurde hinsichtlich einer Inhibierung der morphologischen Veränderung in CHO-Zellen untersucht. Das getestete Pertussis-Holotoxin vom Wildtyp und das getestete rekombinante Pertussis-Holotoxin wurden auf etwa entsprechende Konzentrationen der Untereinheit S1 eingestellt, was durch vergleichende Western-Blot-Analyse bestätigt wurde. Es konnte klar gezeigt werden (**Figur 4 B und Figur 4 C**), daß rekombinantes PT denselben morphologischen Effekt wie PT-Holotoxin liefert, das von B. pertussis isoliert wurde (**Figur 4 E und Figur 4 F**), und daß diese Aktivität spezifisch durch den monoklonalen Antikörper E19 inhibiert wird (**Figur 4 D und Figur 4 G**). Da PT-Untereinheiten CHO-Zellen nicht klustern können (1, 3), zeigt dieses Ergebnis, daß im Periplasma von *B. bronchiseptica* ATCC10580::TnfusPT rekombinantes Pertussis-Holotoxin (und nicht nur die Untereinheiten) gebildet wird.

Nachstehend werden die Figuren noch näher erläutert.

**Figur 1**. Es wird die Konstruktion von Suicidvektoren gezeigt, die Mini-Transposons für PT-Expression unter der Kontrolle des tac-Promotors (pMW126) aufweisen bzw. für Transkriptionsfusionen mit nativen Promotoren (pMW127) vorgesehen sind. Das PT-Operon wurde aus Plasmid pTX42 (22) kloniert. Die PT-Gene für die Untereinheiten S1 bis S5 sind durch weiße Pfeile wiedergegeben. Die Plasmide pUT::miniTn5/Km und pUT::TnPtac enthalten die IS50_{R}-Transposase (tnp; langer schattierter Balken) und 19 Basenpaare vom terminalen Tn5-Ende (kurze schattierte Balken), die das Kanamycinresistenzgen (Kan=Km^{r}; weißer Balken) von pUT::miniTn5/Km flankieren, bzw. das Kanamycinresistenzgen, das Laktoserepressorgen (LacI^{q}) und den tac-Promotor (P_{tac}; schwarzer Pfeil) von pUT::TnPtac flankieren. Die terminalen Tn5-Enden bezeichnen die Mini-Transposons TnPtacPT (pMW126) und TnfusPt (pMW127). Das Suicidvehikel pUT enthält auch eine mob-Stelle (mob; schwarzer Balken), die einen konjugalen Transfer kodiert, sowie das Ampicillinresistenzgen (Bla,; weißer Balken) zur Selektion. Die Richtung der Transkription ist entweder durch Pfeile oder durch die Orientierung der weißen Pfeile angegeben. Die Relationen der Merkmale der dargestellten Plasmide sind nicht getreu wiedergegeben, auch sind nur relevante Restriktionsstellen dargestellt.

**Figur 2.** Es wird eine Southern-Hybridizationsanalyse von *B. bronchiseptica* ATCC10580 gezeigt, wobei dieser Stamm PT-exprimierende Mini-Transposons enthält.
A Zeile 1: Diagramm für das PT-Operon.
A Zeile 2: Diagramm für TnPtacPt
A Zeile 3: Diagramm für TnfusPt
Bezüglich der Symbole vergleiche man Figur 1. Nur die EcoRI-Restriktionsstellen sind dargestellt.
B: Southern-Blot (unter Verwendung von pMW126 als Probe) für chromosomale DNA, die mit EcoRI verdaut und von

| | |
|---|---|
| ATCC10580 | (Streifen 1) |
| ATCC10580::TnPtacPT bzw. | (Streifen 2) |
| ATCC10580::TnfusPT isoliert wurde | (Streifen 3) |
| DNA des Plasmids pMW126, die mit EcoRI verdaut worden war, ist gleichfalls dargestellt | (Streifen 4) |

Molekulargewichtsmarker (Größe in Kilobasen = kb) und der Ursprung der Reaktionsbanden (vergl. A) sind durch Pfeile markiert.

**Figur 3.** Es sind die Analysenergebnisse einer PT-Expression von

| | |
|---|---|
| *B. bronchiseptica* ATCC10580 | (Streifen 1) |
| mit einem Gehalt an TnPtacPt | (Streifen 2) |
| bzw. TnfusPt wiedergegeben | (Streifen 3). |
| | |
| Ferner sind *B. pertussis* Tohama | (Streifen 4) |
| und gereinigtes PT dargestellt | (Streifen 5) |

A: Gel von Gesamtzellextrakten, das mit Coomassie angefärbt worden war.
B: Western-Blot-Analyse von Gesamtzellextrakten unter Verwendung von spezifischen monoklonalen Anti-PT-Antikörpern, und zwar E19, E205 und E251. Die Positionen der PT-Untereinheiten S1 bis S5 sind durch Pfeile bezeichnet.

**Figur 4.** Charakterisierung von rekombinantem PT.
A: Western-Blot-Analyse von rekombinantem PT, das nach einer Heparin-Sepharose-Chromatographie von periplasmatischen Extrakten von *B. bronchiseptica* ATCC10580::TnfusPT (Streifen 1) eluiert wurde und von gereinigtem PT vom Wildtyp (Streifen 2). Die Positionen der PT-Untereinheiten S1 bis S5 sind durch Pfeile bezeichnet.
B - G: CHO-Klusterassays ohne PT (B und E), mit rekombinatem PT (C und D) und mit Wildtyp-PT (F und G). Monoklonaler Antikörper E19 ist auch D und G zu entnehmen.

### References

1. **Bartley, T. D., D. W. Whiteley, V. L. Mar, D. L. Burns, and W. N. Burnette.** 1989. Pertussis holotoxoid formed *in vitro* with a genetically deactivated S1 subunit. Proc. Natl. Acad. Sci. USA. **86**:8353-8357.
2. **Burnette, W. N., V. L. Mar, W. Cieplak, C. F. Morris, K. T. Kaljot, K. S. Marchitto, R. K. Sachdev, C. Locht, and J. M. Keith.** 1988. Direct expression of *Bordetella pertussis* toxin subunits to high Levels in *Escherichia coli.* Biotechnol. **6**:699-706.
3. **Burns, D. L., J. G. Kenimer, and C. R. Manclark.** 1987. Role of the A subunit of pertussis toxin in alteration of Chinese hamster ovary cell morphology. Infect. Immun. **55**: 24-28.
4. **De Lorenzo, V., M. Herrero, U. Jakubzik, and K. N. Timmis.** 1990. Mini-Tn5 transposon derivatives for insertion mutagenesis, promotor probing, and chromosomal insertion of cloned DNA in Gram-negative eubacteria. J. Bacteriol. **172**:6568-6572.
5. **Gross, R., and R. Rappuoli.** 1988. Positive regulation of pertussis toxin expression. Proc. Natl. Acad. Sci. **85**:3913-3917.
6. **Herrero, M., V. De Lorenzo, and K. N. Timmis.** (1990) Transposon vectors containing non-antibiotic resistance selection markers for cloning and stable chromosomal insertion of foreign genes in Gram-negative bacteria. J. Bacteriol. **172**: 6557-6567.
7. **Keen, N. T., S. Tamaki, D. Kobayashi, and D. Trollinger.** 1988. Improved broad-hostrange plasmids for DNA cloning in Gram-negative bacteria. Gene **70**:191-197.
8. **Kimura, A., K. T. Mountzouros, P. A. Schad, W. Cieplak, and J. L. Cowell.** 1990. Pertussis toxin analog with reduced enzymatic and biological activities is a protective antigen. Infect. Immun. **58**:3337-3347.
9. **Knapp, S., and J. J. Mekalanos.** 1988. Two *trans*-acting regulatory genes (*vir* and *mod*) control antigenic modulation in *Bordetella pertussis.* J. Bacteriol. **170**:5059-5066.
10. **Lee, C. K., A. Roberts, and S. Perrin.** 1989. Expression of pertussis toxin in *Bordetella bronchiseptica* and *Bordetella parapertussis* carrying recombinant plasmids. Infect. Immun. **57**:1413-1418.
11. **Locht, C., P. A. Barstad, J. E. Coligan, L. Mayer, J. J. Munoz, S. G. Smith, and J. M. Keith.** 1986. Molecular cloning of pertussis toxin genes. Nucl. Acids Res. **14**:3251-3261.
12. **Megret, F., and J. E. Alouf.** (1988). A simple novel approach for the purification of pertussis toxin. FEMS Microbiol. Letts. **51**:159-162.
13. **Monack, D. M., B. Arico, R. Rappuoli, and S. Falkow.** 1989. Phase variants of *Bordetella bronchiseptica* arise by spontaneous deletions in the *vir* locus. Mol. Microbiol. **12**:1719-1728.
14. **Nencioni, L., M. Pizza, M. Bugnoli, T. de Magistris, A. di Tommaso, F. Giovannoni, R. Manetti, I. Marsili, G. Matteucci, D. Nucci, R. Oliviieri, P. Pileri, R. Presentini, L. Villa, J. G. Kreeftenberg, S. Silvestri, A. Tagliabue, and R. Rappuolli.** 1990. Characterization of genetically inactivated pertussis toxin mutants: candidates for a new vaccine against whooping cough. Infect. Immun. **58**:1308-1315.
15. **Robinson, A., L. I. Irons, and L. A. E. Ashworth.** 1985. Pertussis vaccine: present status and future prospects. Vaccine **3**:11-22.
16. **Robinson, A., and L. A. E. Ashworth.** 1988. Acellular and defined-component vaccines against pertussis, p. 399-417. *In* A. C. Wardlaw, and R. Parton (ed.), Pathogenesis and Immunity in Pertussis. John Wiley and Sons, Chichester.
17. **Walker, M. J., R. Rohde, R. M. Brownlie, and K. N. Timmis.** 1990. Engineering upstream transcriptional and translational signals of *Bordetella pertussis* serotype 2 fimbrial subunit protein for efficient expression in *Escherichia coli: in vitro* autoassembly of the expressed product into filamentous structures. Mol. Microbiol. **4**.39-47.
18. **Weiss, A. A., and S. Falkow.** 1984. Genetic analysis of phase change in *Bordetella pertussis.* Infect. Immun. **43**:263-269.
19. **Witvliet, M. H., D. L. Burns, M. J. Brennan, J. T. Poolman, and C. R. Manclark.** 1989. Binding of pertussis toxin to eucaryotic cells and glycoproteins. Infect. Immun. **57**:3324-3330.

## Patentansprüche

1. Plasmid mit einem Hybridtransposon für eine stabile Expression großer Mengen von Pertussis-Toxin, das für Vaccine verwendet werden kann, wobei das Transposase-Gen außerhalb der Umkehrwiederholungen des Transposons angeordnet ist und das Plasmid dadurch herstellbar ist, daß man ein Pertussis-Toxin-Operon (PT-Operon) zwischen die Umkehrwiederholungen des Transposons einsetzt, wobei das Plasmid eine stabile Insertion des Pertussis-Toxin-Operons in einen bvg-negativen Stamm ermöglicht.

2. Plasmid nach Anspruch 1, dadurch herstellbar, daß man die Umkehrwiederholungen ungekürzt oder gekürzt verwendet, wobei die gekürzten Umkehrwiederholungen noch eine Transponierung gestatten.

3. Plasmid nach Anspruch 1 oder 2, dadurch **gekennzeichnet**, daß es sich bei dem Transposon um ein Mini-Transposon handelt.

4. Plasmid nach einem der vorhergehenden Ansprüche, dadurch **gekennzeichnet**, daß das Transposon auf Tn5 zurückgeht und vorzugsweise Umkehrwiederholungen einer Länge von jeweils etwa 19 Basenspaaren übernommen worden sind.

5. Plasmid nach einem der vorhergehenden Ansprüche, dadurch herstellbar, daß man ein PT-Operon einbaut, bei dem es sich um ein PT-Operon vom Wildtyp handelt oder um ein PT-Operon, das eine Pertussis-Toxin-Modifikation kodiert, die zur Keuchhustenbekämpfung einsetzbar ist.

6. Plasmid nach einem der vorhergehenden Ansprüche, dadurch **gekennzeichnet**, daß das Hybridtransposon ein Resistenzgen umfaßt, vorzugsweise das Kanamycinresistenzgen (Km^{r} = Kan).

7. Plasmid nach Anspruch 6, dadurch **gekennzeichnet**, daß das Resistenzgen in derselben oder in entgegengesetzter Leserichtung wie das PT-Operon angeordnet ist.

8. Plasmid nach Anspruch 4 mit einem Hybridtransposon mit den folgenden Merkmalen:
(a) Suizidvektor, der ein Mini-Transposon für PT-Expression unter der Kontrolle des tac-Promotors (P_{tac}) aufweist, enthaltend
- eine mob-Stelle (mob),
- das Ampicillin-Resistenzgen (Bla) und
- jeweils 19 Basenpaare vom terminalen Tn5-Ende, die
- das Kanamycin-Resistenzgen (Kan = Km^{r}),
- das Lactoserepressor-Gen (Lac I^{q}),
- den tac-Promotor (Ptac) und
- das PT-Operon (S1, S2, S4, S5, S3) flankieren, sowie
- das IS50_{R}-Transposase-Gen (tnp)
oder
(b) Suizidvektor, der ein Mini-Transposon für PT-Expression mit nativen Promotoren nach Transkriptionsfusion aufweist, mit
- einer mob-Stelle (mob),
- dem Ampicillin-Resistenzgen (Bla) und
- jeweils 19 Basenpaaren vom terminalen Tn5-Ende, die
- das Kanamycin-Resistenzgen (Kan = Km^{r}) und
- das PT-Operon (S1, S2, S4, S5, S3) flankieren, sowie
- dem IS50_{R}-Transposase-Gen (tnp).

9. Bvg-negativer Bakterienstamm mit einem Plasmid gemäß einem der vorhergehenden Ansprüche, vorzugsweise Bordetella pertussis, Bordetella parapertussis oder Bordetella bronchiseptica, insbesondere ATTC 10580.

10. Bvg-negativer Bakterienstamm, gewinnbar durch Einbau eines PT-Operons mit Hilfe eines Plasmids gemäß einem der Ansprüche 1 bis 8 in Bordetella pertussis, Bordetella parapertussis oder Bordetella bronchiseptica, insbesondere ATTC 10580.

11. Verwendung eines bvg-negativen Bakterienstamms gemäß Anspruch 9 oder 10 zur stabilen Expression großer Mengen von Pertussis-Toxin für Vaccine.

## Claims

1. Plasmid comprising a hybrid transposon for a stable expression of large amounts of pertussis toxin which can be used for vaccines, wherein the transposase gene is positioned outside of the inverted repeats of the transposon and wherein the plasmid can be produced by inserting the pertussis toxin operon (PT operon) in between the inverted repeats of the transposon, wherein the plasmid enables a stable insertion of the pertussis toxin operon into a bvg negative strain.

2. Plasmid according to claim 1, which can be produced by using the inverted repeats in unshortened or shortened form, the shortened inverted repeats permitting a further transposition.

3. Plasmid according to claim 1 or claim 2, **characterized** in that the transposon is a mini-transposon.

4. Plasmid according to anyone of the preceding claims, **characterized** in that the transposon is derived from Tn5 and that preferably inverted repeats each having a length of approximately 19 base pair have been taken.

5. Plasmid according to anyone of the preceding claims which can be produced by incorporating a PT operon which is a wild-type PT operon or a PT operon that encodes a pertussis toxin modification that can be used for combating whooping cough.

6. Plasmid according to anyone of the preceding claims, **characterized** in that the hybrid transposon comprises a resistance gene, preferably the kanamycin resistance gene (Km^{r} = Kan).

7. Plasmid according to claim 6, **characterized** in that the resistance gene is arranged in the same direction of reading as or in the opposite direction of reading to the PT operon.

8. Plasmid according to claim 4 comprising a hybrid transposon having the following features:
(a) Suicide vector which comprises a mini-transposon for a PT expression under the control of the tac-promotor (P_{tac}), containing
- mob side (mob),
- the ampicillin resistance gene (Bla) and
- 19 base pairs each of the terminal Tn5 terminus which flank
- the kanamycin resistance gene (Kan = Km^{r}),
- the lactose repressor gene (Lac I^{q}),
- the tac-promotor (Ptac) and
- the PT-operon (S1, S2, S4, S5, S3) and, in addition,
- the IS50_{R} transposase gene (tnp)
or
(b) suicide vector comprising a mini-transposon for a PT expression by means of native promotors after transcription fusion comprising
- a mob side (mob)
- the ampicillin resistance gene (Bla) and
- 19 base pairs each of the terminal Tn5 terminus which flank
- the kanamycin resistance gene (Kan = Km^{r}), and
- the PT-operon (S1, S2, S4, S5, S3) and, in addition,
- the IS50_{R} transposase gene (tnp).

9. Bvg negative bacterial strain comprising a plasmid according to anyone of the preceding claims, preferably Bordetella pertussis, Bordetella parapertussis or Bordetella bronchiseptica, especially ATCC 10580.

10. Bvg negative bacterial strain, which can be obtained by inserting a PT operon by means of a plasmid according to anyone of claims 1 to 8 into Bordetella pertussis, Bordetella parapertussis or Bordetella bronchiseptica, especially ATCC 10580.

11. Use of a bvg negative strain according to claim 9 or 10 for a stable expression of large amounts of pertussis toxin for vaccines.

## Revendications

1. Plasmide comportant un transposon hybride permettant l'expression stable de grandes quantités de toxine coquelucheuse (ou toxine Pertussis) que l'on peut utiliser comme vaccin, le gène de transposase étant situé en dehors des répétitions inversées du transposon et le plasmide pouvant être préparé par insertion d'un opéron "toxine Pertussis" (opéron tP) entre les répétitions inversées du transposon, le plasmide rendant possible une insertion stable de l'opéron tP chez une souche Bvg-négative.

2. Plasmide conforme à la revendication 1, qu'on peut préparer en utilisant des répétitions inversées raccourcies ou non, les répétitions inversées raccourcies permettant encore une transposition.

3. Plasmide conforme à la revendication 1 ou 2, caractérisé en ce que le transposon est un mini-transposon.

4. Plasmide conforme à l'une des revendications précédentes, caractérisé en ce que le transposon dérive de Tn5 et comporte, de préférence, des répétitions inversées longues chacune d'environ 19 paires de bases.

5. Plasmide conforme à l'une des revendications précédentes, qu'on peut préparer par incorporation d'un opéron tP qui est un opéron tP de type sauvage ou un opéron tP qui code une variante de la toxine Pertussis que l'on peut utiliser pour lutter contre la coqueluche.

6. Plasmide conforme à l'une des revendications précédentes, caractérisé en ce que le transposon hybride englobe un gène de résistance, de préférence un gène de résistance à la kanamycine (Km^{r} = Kan).

7. Plasmide conforme à la revendication 6, caractérisé en ce que le gène de résistance est disposé dans le même sens de lecture que l'opéron tP ou en sens contraire.

8. Plasmide conforme à la revendication 4, comportant un transposon hybride présentant les caractéristiques suivantes :
a) vecteur suicide qui présente un mini-transposon pour l'expression de toxine Pertussis sous le contrôle du promoteur tac (P_{tac}), contenant :
- un site mob (mob),
- le gène de résistance à l'ampicilline (Bla), et
- les extrémités de Tn5, de 19 paires de bases chacune, flanquant
- le gène de résistance à la kanamycine (Kan = Km^{r}),
- le gène du répresseur lactose (Lac I^{q}),
- le promoteur tac (P_{tac}), et
- l'opéron tP (S1, S2, S4, S5, S3), ainsi que
- le gène de transposase d'IS50_{R} (tnp),
ou bien
b) vecteur suicide qui présente un mini-transposon pour l'expression de toxine Pertussis à l'aide de promoteurs naturels après transcription-fusion, comportant :
- un site mob (mob),
- le gène de résistance à l'ampicilline (Bla), et
- les extrémités de Tn5, de 19 paires de bases chacune, flanquant
- le gène de résistance à la kanamycine (Kan = Km^{r}), et
- l'opéron tP (S1, S2, S4, S5, S3), ainsi que
- le gène de transposase d'IS50_{R} (tnp).

9. Souche bactérienne Bvg-négative contenant un plasmide conforme à l'une des revendications précédentes, qui est de préférence une souche Bordetella pertussis, Bordetella parapertussis ou Bordetella bronchiseptica, en particulier ATCC 10580.

10. Souche bactérienne Bvg-négative, que l'on peut obtenir en introduisant, à l'aide d'un plasmide conforme à l'une des revendications 1 à 8, un opéron tP chez Bordetella pertussis, Bordetella parapertussis ou Bordetella bronchiseptica, en particulier ATCC 10580.

11. Emploi d'une souche bactérienne Bvg-négative conforme à la revendication 9 ou 10 pour produire de manière stable, par expression génique, de grandes quantités de toxine Pertussis destinée à des vaccins.
